# EUROPEAN PATENT APPLICATION

(11) **EP 2 436 301 A1**
(43) Date of publication of application: **04.04.2012**
(21) Application number: 11007671.8
(22) Date of filing: 21.09.2011
(51) Int. Cl.: A61B 1/04, A61B 1/05, A61B 1/06

(54) **Illumination apparatus and examination system**

(30) Priority: 30.09.2010 JP 2010222669
(71) Applicant: OLYMPUS CORPORATION, Shibuya-ku Tokyo 151-0072 (JP)
(72) Inventor: Kumai, Katsunori, Tokyo 151-0072 (JP)
(74) Representative: Schicker, Silvia

(57) **Abstract**

Provided is an illumination apparatus that can switch between at least two illumination modes having different spectral characteristics in a short period of time.

The illumination apparatus includes light sources 1 and 2 configured to generate light in wavelength bands required for at least two illumination modes; a dichroic mirror 5 configured to transmit light in a predetermined band from the light source 1, to reflect light from the light source 2 outside the predetermined band in the direction of the optical axis of light from the light source 1, and to combine the light from the light source 1 and the light from the light source 2; and a control unit configured to control the illumination states of the light sources 1 and 2.

## Description

### {Technical Field}

The present invention relates to an illumination apparatus and an examination system including the same.

### {Background Art}

There is a known illumination apparatus that is provided with a movable filter, which is constituted of a filter having at least two different spectral characteristics, interposed between a white light source and an illuminated area and that moves the filter depending on an illumination mode to mechanically switch the light to be transmitted through the illuminated part, from the white light (for example, refer to PTL 1).
Another known illumination apparatus has a brightness priority mode and color priority mode and outputs a mode setting signal to electrically switch between the brightness priority mode and the color priority mode (for example, refer to PTL 2).
Another known illumination apparatus partially replaces main illumination light from a lamp, etc., which is substantially white, with sub-illumination light from a laser light source, etc. and enhances the emission spectrum of the main illumination light with the sub-illumination light to generates illumination light (for example, see PTL 3).
{Citation List}
{Patent Literature}

{PTL 1} Japanese Unexamined Patent Application, Publication No. 2001-314370
{PTL 2} Japanese Unexamined Patent Application, Publication No. 2006-349731
{PTL 3} Japanese Unexamined Patent Application, Publication No. 2002-296680

### {Summary of Invention}

### {Technical Problem}

When examination or measurement of a subject is to be carried out in at least two illumination modes with different spectral characteristics, a reduction in the switching time is required for reducing the time required for the operation. Moreover, during examination, it is required that examination in multiple illumination modes be repeated in a short amount of time to make it easier to confirm the position of particular sites by displaying two screens on a video monitor or by overlaying two screens. Specifically, an example of endoscopy includes fluoroscopy, etc. that identifies the position of an affected area by overlaying the affected area, which emits fluorescence of a predetermined wavelength, on an examination image obtained with white illumination light.

Unfortunately, with the switching method disclosed in PTL 1, because the filters are mechanically moved, it takes some time to switch the filters, and thus, the time required for the operation increase. Moreover, if the boundary regions of different filters are disposed in the optical path during switching, the problem of image distortion occurs.

With the switching method disclosed in PTL 2, the driving conditions of an LED can be changed depending on the illumination mode to perform instantaneous mode switching. However, one problem is that the desired spectral characteristic often cannot be acquired because only the color balance of the illumination apparatus, which is constituted of individual RGB light sources, can be changed.

PTL 3 discloses an illumination apparatus in which there is illumination-light combining means for enhancing a predetermined wavelength band of an emission spectrum of main illumination light with sub-illumination light and generating illumination light by replacing the main illumination with the sub-illumination light in a predetermined wavelength band in which the light intensity of the main illumination light is smaller than that of the sub-illumination light from a light source; however, there is no disclosure associated with the switching of illumination modes, and thus, inconveniently, in some cases, the desired spectral characteristic cannot be acquired.

The present invention has been conceived in light of the circumstances described above, and an object thereof is to provide an illumination apparatus and an examination system that can switch between at least two illumination modes having different spectral characteristics.

### {Solution to Problem}

To achieve the object described above, the present invention provides the following solutions.
A first aspect of the present invention is an illumination apparatus configured to illuminate an illumination area in a plurality of illumination modes constituted of light in different predetermined bands, having bands in which a first band required by one illumination mode of the plurality of illumination modes is narrower than a second band required by another illumination mode, the apparatus including a first light source configured to emit illumination light in a band substantially the same as the first band; a second light source configured to emit illumination light in at least the second band; a light combining unit configured to transmit light in the first band, among the illumination light emitted from the first light source, in the direction of the illumination area as transmitted light while reflecting light outside the first band, among the illumination light emitted from the second light sources, in the direction of the illumination area as reflected light and to optically combine the transmitted light and the reflected light; an illumination-mode selecting unit configured to select an illumination mode from the plurality of illumination modes; and a control unit configured to control the light intensity of light emitted from the first light source and the second light source on the basis of the selected illumination mode, wherein the one illumination mode is a first illumination mode for lighting only the first light source, and the other illumination mode is a second illumination mode for lighting both the first light source and the second light source.

A second aspect, which is described below and differs from the first aspect in that the positions of the light sources with respect to the light combining unit differ, may be employed.
A second aspect of the present invention is an illumination apparatus configured to illuminate an illumination area in a plurality of illumination modes constituted of light in different predetermined bands, having bands in which a first band required by one illumination mode of the plurality of illumination modes is narrower than a second band required by another illumination mode, the apparatus including a first light source configured to emit illumination light in a band substantially the same as the first band; a second light source configured to emit illumination light in at least the second band; a light combining unit configured to reflect light in the first band, among the illumination light emitted from the first light source, in the direction of the illumination area as reflected light while transmitting light outside the first band, among the illumination light emitted from the second light sources, in the direction of the illumination area as transmitted light and to optically combine the transmitted light and the reflected light; an illumination-mode selecting unit configured to select an illumination mode from the plurality of illumination modes; and a control unit configured to control the light intensity of light emitted from the first light source and the second light source on the basis of the selected illumination mode, wherein the one illumination mode is a first illumination mode for lighting only the first light source, and the other illumination mode is a second illumination mode for lighting both the first light source and the second light source.

According to the first and second aspects of the present invention, illumination light in a wavelength band corresponding to the illumination mode can be provided by controlling the light intensity of the light emitted from the first light source and the second light source in accordance with the illumination mode and optically combining the light emitted from the first light source and the second light source.

In the plurality of illumination modes, the first band required by one illumination mode in the plurality of illumination modes is narrower than a second band required by another illumination mode, and the first light source emits illumination light in a band substantially the same as the first band. The second light source emits illumination light in at least the second band. That is, the illumination light emitted from the second light source has a band wider than that of the illumination light emitted from the first light source.
Therefore, when the first light source and the second light sources are simultaneously illuminated, light in mutually complementary wavelength bands is emitted as combined light. When only the first light source is lit, illumination light in a band substantially the same as the first band is emitted as combined light.

Accordingly, for example, by lighting all light sources in a second illumination mode, regular examination can be performed by irradiating a subject with light in a wide band (for example, white light). Moreover, for example, by lighting the first light source in a first illumination mode, special-light examination can be performed by irradiating the subject with light in a narrow band (for example, blue light). In this way, by controlling the illumination state of the first light source and the second light source, the examination mode can be switched instantaneously.

A third embodiment of the present invention in examination system including one of the illumination apparatus described above; an irradiation optical system configured to irradiate a subject with light combined at the light combining unit; and a light receiving unit configured to receive light reflected at the subject, wherein the control unit controls the emission light intensity of the first light source and the second light source on the basis of the intensity of the reflected light received by the light receiving unit.

According to the third aspect of the present invention, the subject is irradiated with the light combined at the light combining unit by the irradiation optical system, and the light reflected at the subject is received by the light receiving unit. Then, the control unit controls the emission light intensity of the first light source and the second light source on the basis of the intensity of the reflected light received by the light receiving unit. In this way, the intensity of the light emitted toward the subject can be adjusted in response to the intensity of the light reflected at the subject, and the examination precision of the subject can be improved.

In the third aspect described above, the light receiving unit may be an image acquisition element configured to acquire an image of the subject, and the control unit may control the emission light intensity of the first light source and the second light source so as to set the brightness of the image acquired by the image acquisition element constant.
In this way, an image of the subject can be acquired by the image acquisition element, the emission light intensity of the first light source and the second light source can be controlled such that the brightness of the image is constant, and the subject can be examined at a constant brightness.

### {Advantageous Effects of Invention}

The present invention is advantageous in that at least two illumination modes having different spectral characteristics can be switched between in a short amount of time.

### {Brief Description of Drawings}

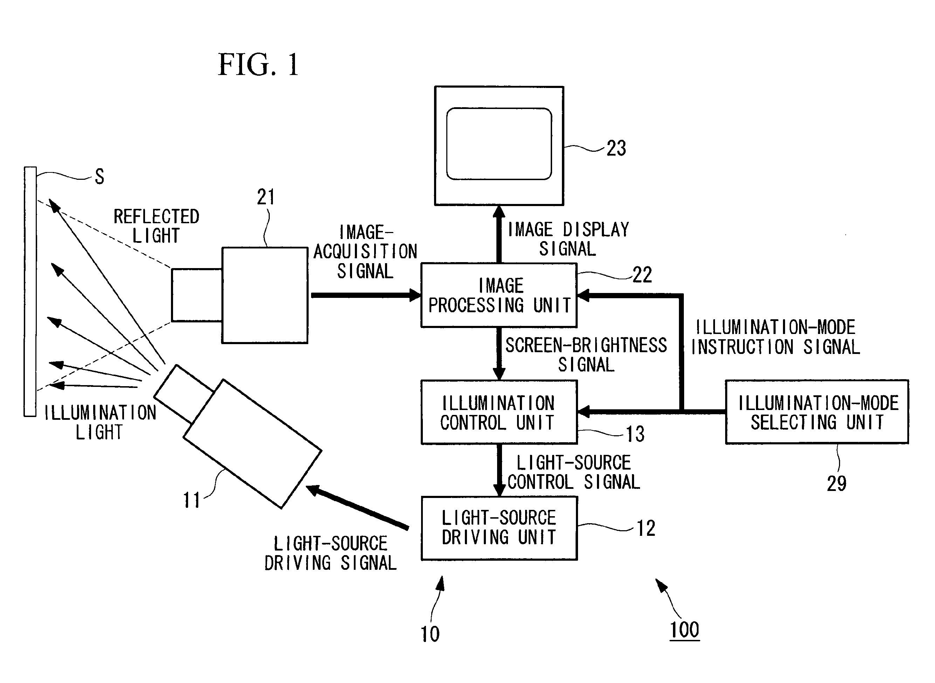

{Fig. 1}
   Fig. 1 is a schematic configuration diagram of an examination system according to embodiments of the present invention.
{Fig. 2}
   Fig. 2 is a schematic configuration diagram of an illumination apparatus according to a first embodiment of the present invention.
{Fig. 3}
   Fig. 3 is a graph illustrating the spectral characteristic of a first light source in Fig. 2.
{Fig. 4}
   Fig. 4 is a graph illustrating the spectral characteristic of a second light source in Fig. 2.
{Fig. 5}
   Fig. 5 is a graph illustrating the reflection characteristic of a dichroic mirror in Fig. 2.
{Fig. 6}
   Fig. 6 illustrates the illumination state of each light source in each illumination mode of the illumination apparatus in Fig. 2.
{Fig. 7}
   Fig. 7 is a graph illustrating the spectral characteristic of illumination light emitted from the illumination apparatus in Fig. 2 in a white light mode.
{Fig. 8}
   Fig. 8 is a graph illustrating the spectral characteristic of illumination light emitted from the illumination apparatus in Fig. 2 in a special light mode.
{Fig. 9}
   Fig. 9 is a schematic configuration diagram of an illumination apparatus according to a second embodiment of the present invention.
{Fig. 10}
   Fig. 10 is a graph illustrating the spectral characteristic of a first light source in Fig. 9.
{Fig. 11}
   Fig. 11 is a graph illustrating the spectral characteristic of a second light source in Fig. 9.
{Fig. 12}
   Fig. 12 is a graph illustrating the reflection characteristic of a dichroic mirror in Fig. 9.
{Fig. 13}
   Fig. 13 is a diagram illustrating the illumination state of each light source in each illumination mode of the illumination apparatus in Fig. 9.
{Fig. 14}
   Fig. 14 is a graph illustrating the spectral characteristic of illumination light emitted from the illumination apparatus in Fig. 9 in a white light mode.
{Fig. 15}
   Fig. 15 is a graph illustrating the spectral characteristic of illumination light emitted from the illumination apparatus in Fig. 9 in a special light mode.
{Fig. 16}
   Fig. 16 is a schematic configuration diagram of an illumination apparatus according to a third embodiment of the present invention.
{Fig. 17}
   Fig. 17 is a graph illustrating the spectral characteristic of a first light source in Fig. 16.
{Fig. 18}
   Fig. 18 is a graph illustrating the spectral characteristic of a second light source in Fig. 16.
{Fig. 19}
   Fig. 19 is a graph illustrating the reflection characteristic of a first dichroic mirror in Fig. 16.
{Fig. 20}
   Fig. 20 is a graph illustrating the reflection characteristics of second and third dichroic mirrors in Fig. 16.
{Fig. 21}
   Fig. 21 is a graph illustrating the reflection characteristic of a fourth dichroic mirror in Fig. 16.
{Fig. 22}
   Fig. 22 is a graph illustrating the spectral characteristic of light traveling along an optical path A in the illumination apparatus in Fig. 16.
{Fig. 23}
   Fig. 23 is a graph illustrating the spectral characteristic of light traveling along an optical path B in the illumination apparatus in Fig. 16.
{Fig. 24}
   Fig. 24 is a graph illustrating the spectral characteristic of light traveling along an optical path C in the illumination apparatus in Fig. 16.
{Fig. 25}
   Fig. 25 is a graph illustrating the spectral characteristic of light traveling along an optical path D in the illumination apparatus in Fig. 16.
{Fig. 26}
   Fig. 26 is a graph illustrating the spectral characteristic of illumination light emitted from the illumination apparatus in Fig. 16 in a white light mode.
{Fig. 27}
   Fig. 27 is a graph illustrating the spectral characteristic of illumination light emitted from the illumination apparatus in Fig. 16 in a special light mode.

### {Description of Embodiments}

### First Embodiment

An illumination apparatus according to a first embodiment of the present invention will be described below with reference to the drawings.
Fig. 1 is a configuration diagram illustrating, in outline, an examination system 100 including an illumination apparatus according to this embodiment.
As illustrated in Fig. 1, the examination system 100 according to this embodiment includes an illumination apparatus 10 constituted of a light source unit (irradiation optical system) 11, a light-source driving unit 12, and an illumination control unit (control unit) 13; an image acquisition unit (light receiving unit) 21; an image processing unit 22; and an image monitor 23.

The light source unit 11 has a plurality of light sources and emits illumination light toward a subject S. The detailed configuration of the light source unit 11 will be described below.
The image acquisition unit 21 is, for example, an image acquisition element, such as a CCD, and detects ight reflected at the subject S. That is, the image acquisition unit 21 functions as light-receiving means for receiving light reflected at the subject S. Moreover, the image acquisition unit 21 acquires an image of the subject S by detecting the light reflected at the subject S and outputting an image acquisition signal to the image processing unit 22.

The image processing unit 22 processes the image acquisition signal output from the image acquisition unit 21 to generate an image of the subject S. The image processing unit 22 outputs the generated image of the subject S to the image monitor 23 as an image display signal and outputs the brightness of the image of the subject S to the illumination control unit 13 as a screen brightness signal.
The image monitor 23 displays the image of the subject S on a monitor screen on the basis of the image display signal output from the image processing unit 22.

The illumination control unit 13 receives a screen brightness signal output from the image processing unit 22, as well as an illumination-mode instruction signal output from the illumination-mode selecting unit 29, such as, for example, a switch. Here, the illumination-mode instruction signal is a signal that instructs the switching of illumination light to be emitted from the light source unit 11 toward the subject S to, for example, white light or special light.

The illumination-mode instruction signal is also sent to the image processing unit 22, where an image may be generated by switching to an image processing method optimal for each illumination mode. For example, in an examination method of forming a clear image of blood vessels by irradiating them with only light near 415 nm and near 540 nm, which are easily absorbed by hemoglobin in blood, reflected light near 415 nm is generated as B (blue) and G (green) channel signals of the displayed image, and reflected light near 540 nm is generated as an R (red) channel signal of the displayed image, and these signals are output to the image monitor 23.

The illumination control unit 13 generates a light-source control signal on the basis of the screen brightness signal output from the image processing unit 22 and outputs this signal to the light-source driving unit 12. Specifically, the illumination control unit 13 calculates the light intensity of the illumination light emitted from the light source unit 11 such that the brightness of the image of the subject S generated by the image processing unit 22 is constant and outputs this to the light-source driving unit 12 as a light-source control signal. Moreover, in response to the selected illumination mode, the illumination control unit 13 outputs to the light-source driving unit 12 a light-source control signal indicating which light source, among the multiple light sources in the light source unit 11, is to be lit.

When the brightness of the subject S cannot be set constant by controlling it with the light-source driving unit 12, the display gain may be set at the image processing unit 22 such that the brightness of the image of the subject S becomes constant when the image of the subject S is displayed on the image monitor 23.

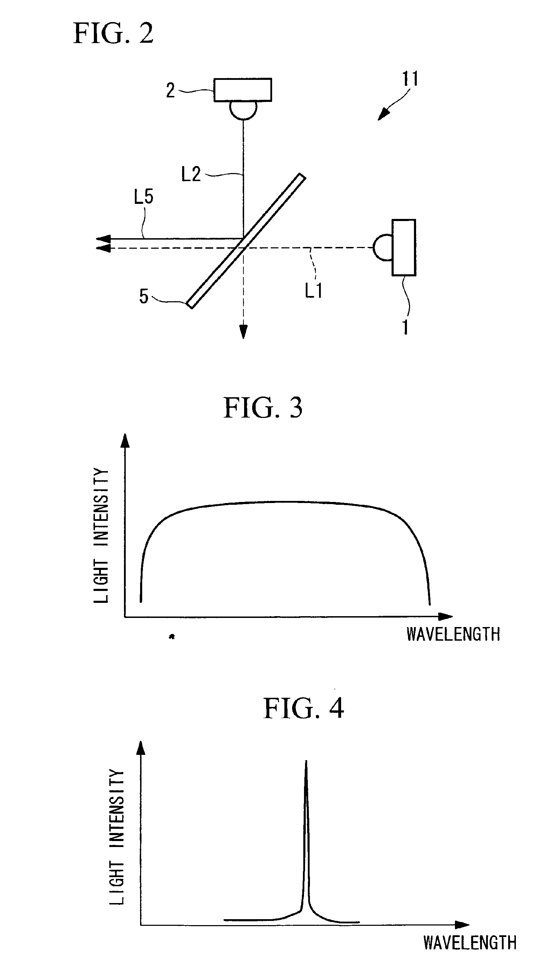

The light-source driving unit 12 drives multiple light sources in the light source unit 11 on the basis of the light-source control signal output from the illumination control unit 13.
As illustrated in Fig. 2, the light source unit 11 includes a light source (first light source) 1 and a light source (second light source) 2, which are disposed such that optical axes thereof are orthogonal to each other, and a dichroic mirror (light combining unit) 5 disposed at the intersection of the optical axis of the light source 1 and the optical axis of the light source 2.

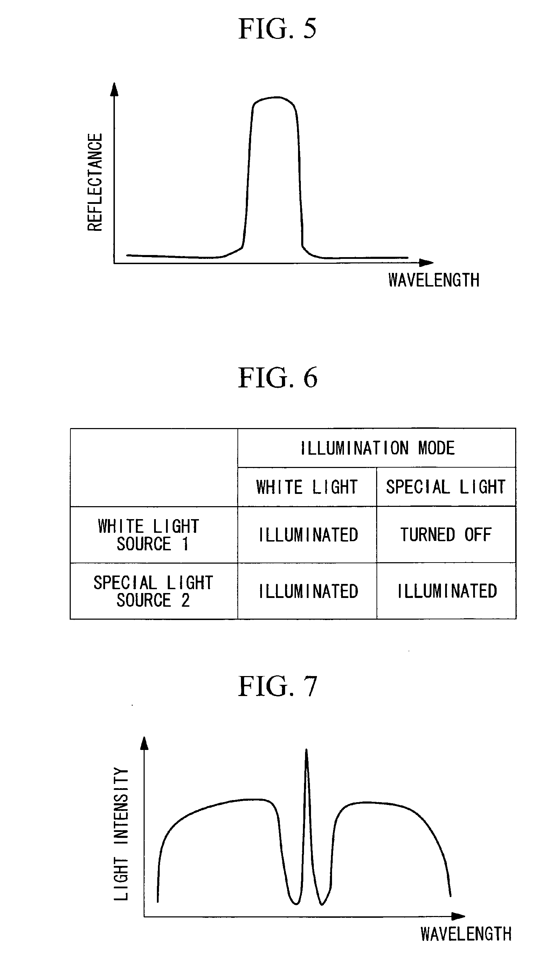

As illustrated in Fig. 3, the light source 1 is a light source that emits white light L1 in a wide wavelength band.
As illustrated in Fig. 4, the light source 2 is a light source that emits light L2 in a narrow wavelength band, such as a laser beam, and emits light having substantially all components in an extremely limited wavelength band (wavelength band of wavelengths λ1 to λ2 illustrated in Fig. 5) compared with the light L1.

As illustrated in Fig. 5, the dichroic mirror 5 has a reflection characteristic in which light in the wavelength bands smaller than wavelength λ1 and larger than or equal to wavelength λ2 is transmitted, white light in the wavelength band between wavelengths λ1 to λ2 is reflected.

By having such a reflection characteristic, the dichroic mirror 5 transmits light in the wavelength bands smaller than wavelength λ1 and greater than or equal to λ2, among the light L1 emitted from the light source 1. Meanwhile, the dichroic mirror 5 reflects light in the wavelength band of wavelengths λ1 to λ2, among the light L1 emitted from the light source 1.

Moreover, the dichroic mirror 5 transmits light in the wavelength bands smaller than wavelength λ1 and greater than or equal to wavelength λ2, among the light L2 emitted from the light source 2. Meanwhile, the dichroic mirror 5 reflects, in the direction of the optical axis of the light L1 emitted from the light source 1, light in the wavelength band of wavelengths λ1 to λ2, among the light L2 emitted from the light source 2. Here, as described above, since the light L2 has substantially all components in the wavelength band of wavelengths λ1 to λ2, the dichroic mirror 5 reflects substantially all components of the light L2 in the direction of the optical axis of the light L1.

Now, in a white light mode in which the subject S is irradiated with white light, the light source 1 and the light source 2 are illuminated as illustrated in Fig. 6.
In such a case, the dichroic mirror 5 combines the light transmitted through the dichroic mirror 5, among the light L1 emitted from the light source 1, and light reflected at the dichroic mirror 5, among the light L2 emitted from the light source 2, and emits this combined light L5 in the direction of the optical axis of the light L1. As illustrated in Fig. 7, the combined light L5 has a spectral characteristic resulting from superimposing the light in the wavelength bands smaller than wavelength λ1 and greater than or equal to wavelength λ2, among the light L1 emitted from the light source 1, and light in the wavelength band of wavelengths λ1 to λ2, among the light L2 emitted from the light source 2.

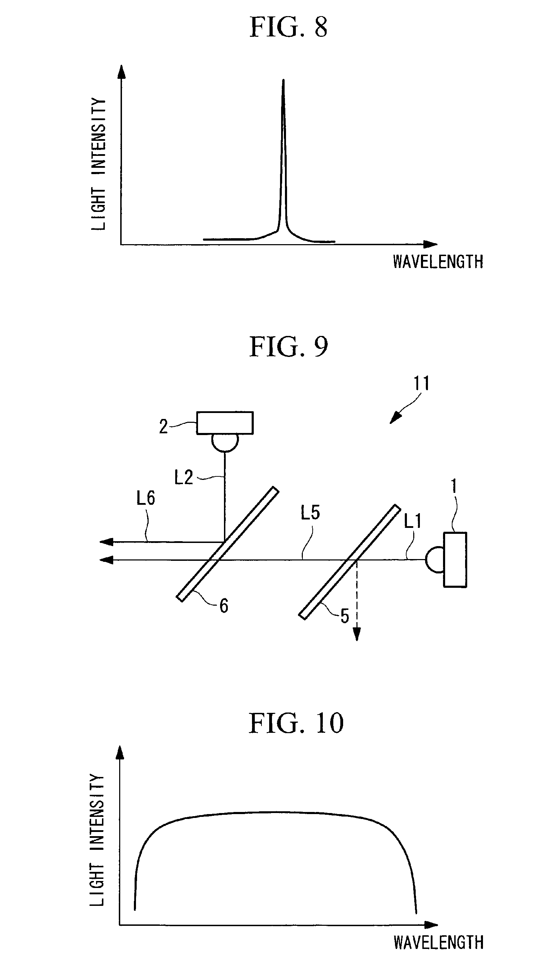

As illustrated in Fig. 6, in a special light mode in which the subject S is irradiated with special light, for example, the light source 1 is turned off, and only the light source 2 is illuminated.
In such a case, the dichroic mirror 5 reflects and emits light in the wavelength band of wavelengths λ1 to λ2, among the light L2 emitted from the light source 2, in the direction of the optical axis of the light L1. In such a case, the spectral characteristic of the light emitted in the direction of the optical axis of the light L1 is as illustrated in Fig. 8.

The operation of the examination system 100 having the above-described configuration will be described below.
First, for example, a user selects an illumination mode from a plurality of illumination modes, and an illumination-mode instruction signal for driving the illumination apparatus 10 in the selected illumination mode is sent from the illumination-mode selecting unit 29, which is not shown.

When the selected illumination mode is the white light mode, the light source 1 and the light source 2 are both driven. In such a case, the dichroic mirror 5 combines the light in the wavelength bands smaller than wavelength λ1 and greater than or equal to wavelength λ2, among the light L1 emitted from the light source 1, and the light of wavelengths λ1 to λ2, among the light L2 emitted from the light source 2, and emits the combined light L5 in the direction of the optical axis of the light L1. As illustrated in Fig. 7, the combined light L5 is white light having a wide wavelength band.

On the other hand, when the selected illumination mode is a special light mode, the light source 1 is turned off, and only the light source 2 is driven. In such a case, the dichroic mirror 5 emits, in the direction of the optical axis of the light L1 emitted from the light source 1, light in the wavelength band of wavelengths λ1 to λ2, among the light L2 emitted from the light source 2. In such a case, as illustrated in Fig. 8, the light emitted in the direction of the optical axis of the light L1 is special light having a narrow wavelength band.

In either illumination mode described above, the light intensity of the illumination light emitted from the light source unit 11 is controlled by the illumination control unit 13 such that the brightness of the image of the subject S is constant. Such control of the light intensity of the illumination light will be described below.
First, the subject S is irradiated with illumination light from the light source unit 11, and the light reflected at the subject S is detected by the image acquisition unit 21.

Next, the image processing unit 22 processes an image acquisition signal output from the image acquisition unit 21 to generate an image of the subject S, and this image of the subject S is displayed on the image monitor 23. Also, the brightness of the image of the subject S is output to the illumination control unit 13 as a screen brightness signal.

At the illumination control unit 13, the light intensity of the illumination light emitted from the light source unit 11 is controlled on the basis of the screen brightness signal from the image processing unit 22 such that the brightness of the image of the subject S is constant. Two cases of intensity control of the illumination light, which is mentioned above, for simply switching the illumination mode and for alternating between two illumination modes in short periods of time will be described below.

When the illumination mode is simply switched, in the white light mode, the light source 1 and the light source 2 are illuminated, and the light intensity of the two light sources is controlled to maintain the color balance while maintaining a constant light intensity ratio of the two light sources such that the brightness of the screen is constant.

In the special light mode, only the light source 2 is illuminated, and the light intensity of the light source 2 is controlled such that the brightness of the screen is constant.
As described above, by controlling the light source 1 and the light source 2, in either the white light mode or the special light mode, the brightness of the image of the subject S displayed on the image monitor 23 can be maintained constant.

When examining an area emitting fluorescence as a result of being excited by a specific wavelength, it is difficult to determine the position of a specific area by examining only a fluorescence image. Thus, the image acquired by using the special light as illumination and an image for comparison acquired by using other illumination light are acquired by alternately switching between the illumination modes in a short period of time, and the two images are displayed side by side or in superimposition.

In the examination system 100 according to this embodiment, the illumination mode is switched every one frame (for example, 1/60 s), and the light emission intensity of the light sources is controlled such that the screen brightness in each illumination mode is constant every time the illumination mode is switched. That is, light intensity control is not performed within a frame, but light intensity control of the light sources is performed on the basis of information about the previous image acquisition screen in the same illumination mode. In this way, the brightness of the image of the subject S displayed on the image monitor 23 can be maintained constant.

As described above, in the illumination apparatus 10 and the examination system 100 according to this embodiment, among the light L1 emitted from the light source 1, light in a predetermined band is transmitted through the dichroic mirror 5. Among the light L2 emitted from the light source 2, substantially all light beams are reflected in the optical axis direction of the light L1 by the dichroic mirror 5. In this way, the light L1 emitted from the light source 1 and the light L2 emitted from the light source 2 are combined and emitted as the combined light L5.

In this way, normal examination can be performed by, for example, lighting the light source 1 and the light source 2 and irradiating the subject S with light in a wide band (for example, white light). Moreover, special examination can be performed by, for example, lighting only the light source 2 and irradiating the subject S with light in a narrow band (for example, blue light).
In this embodiment, it is described that, in the special light mode, the light source 1 is turned off and only the light source 2 is lit; instead, the light source 2 may be turned off and only the light source 1 may be lit.

By switching between the white light mode, the special light mode, etc. with the illumination-mode selecting unit 29, the illumination state of the light sources can be controlled to irradiate the subject S with light corresponding to the examination mode. In this case, since the switching between the two illumination modes having different spectral characteristics can be performed by merely controlling the lighting of the two light sources, the illumination mode switching can be performed instantaneously, and reliability can be improved because movable parts are not included.

Moreover, by acquiring an image of the subject S with the image acquisition unit 21 and controlling the light intensities of the light sources such that the brightness of the image is constant, the light intensity of the light emitted onto the subject S is controlled in response to the intensity of the light reflected at the subject S, and the examination precision of the subject S can be improved. Also, since the illumination light intensity of the light source unit 11 is controlled such that the brightness of the subject S acquired by the image acquisition unit 21 is constant, appropriate screen brightness can be maintained even when the illumination mode is switched.

### Second Embodiment

Next, an illumination apparatus according to a second embodiment of the present invention will be described mainly with reference to Figs. 9 to 15.
The illumination apparatus according to this embodiment differs from the embodiment described above in that two dichroic mirrors are provided. In the following, descriptions of the commonalities with the first embodiment are omitted, and mainly the differences are described.

As illustrated in Fig. 9, a light source unit 11 includes a light source (first light source) 1 and a light source (second light source) 2, which are disposed such that the optical axes thereof are orthogonal to each other, a second dichroic mirror (light combining unit) 6 disposed at the intersection of the optical axis of the light source 1 and the optical axis of the light source 2, and a first dichroic mirror 5 interposed between the light source 1 and the second dichroic mirror 6.

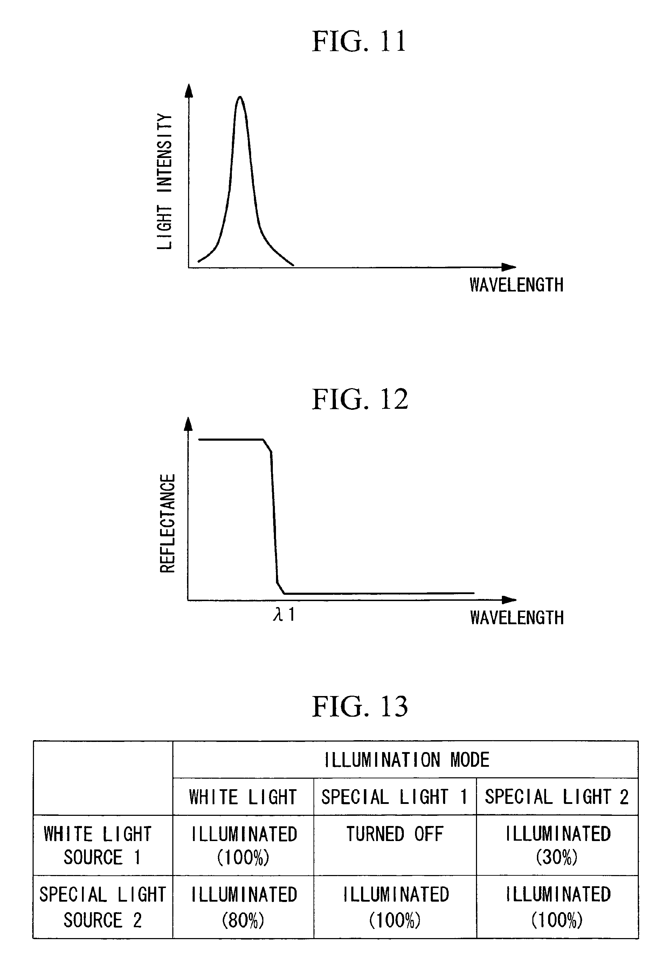

As illustrated in Fig. 10, the light source 1 is a light source that emits white light L1 in a wide wavelength band.
As illustrated in Fig. 11, the light source 2 is a light source that emits light L2 in a narrow wavelength band, such as a laser beam, and emits narrow-band light having substantially all components in an extremely limited wavelength band (wavelength band smaller than wavelength λ1 illustrated in Fig. 12) compared with the light L1.

As illustrated in Fig. 12, the first dichroic mirror 5 and the second dichroic mirror 6 have a reflection characteristic in which light in the wavelength band larger than or equal to wavelength λ1 is transmitted and light in the wavelength band smaller than wavelength λ1 is reflected.

By having such a reflection characteristic, the dichroic mirror 5 transmits light in the wavelength band larger than or equal to wavelength λ1, among the light L1 emitted from the light source 1. Meanwhile, the dichroic mirror 5 reflects light in the wavelength band smaller than wavelength λ1, among the light L1 emitted from the light source 1.

Similar to the dichroic mirror 5, the dichroic mirror 6 transmits light in the wavelength band larger than or equal to wavelength λ1, among the light L1 emitted from the light source 1.
Meanwhile, the dichroic mirror 6 reflects light in the wavelength band smaller than wavelength λ1, among the light L2 emitted from the light source 2, in the direction of the optical axis of the light L1 emitted from the light source 1.

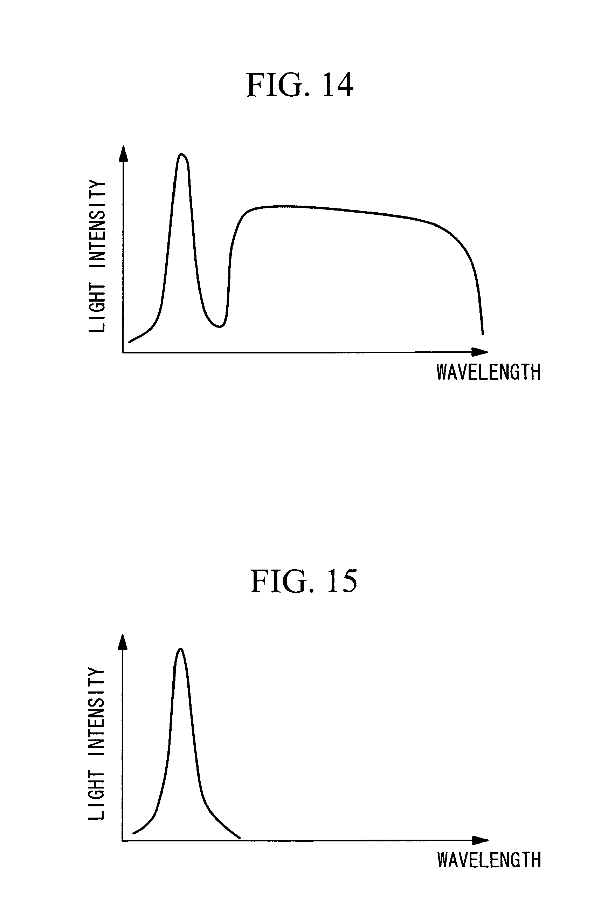

Here, in the white light mode in which the subject S is irradiated with white light, for example, as illustrated in Fig. 13, the light source 1 is lit with 100% intensity, and the light source 2 is lit with 80% intensity.
In such a case, the dichroic mirror 6 combines the light L5 transmitted through the dichroic mirror 5, among the light L1 emitted from the light source 1, and the light reflected at the dichroic mirror 6, among the light L2 emitted from the light source 2, and emits this combined light L6 in the direction of the optical axis of the light L1. As illustrated in Fig. 14, the combined light L6 has a spectral characteristic resulting from superimposing the light in the wavelength band larger than or equal to wavelength λ1, among the light L1 emitted from the light source 1, and light in the wavelength band smaller than wavelength λ1, among the light L2 emitted from the light source 2.

In a special light mode 1 in which the subject S is irradiated with special light, for example, as illustrated in Fig. 13, the light source 1 is turned off, and the only the light source 2 is lit with 100% intensity.
In such a case, the dichroic mirror 6 reflects and emits light in the wavelength band smaller than wavelength λ1, among the light L2 emitted from the light source 2, in the direction of the optical axis of the light L1. In such a case, the spectral characteristic of the light emitted in the direction of the optical axis of the light L1 is as illustrated in Fig. 15.

In a special light mode 2 in which the subject S is irradiated with special light, for example, as illustrated in Fig. 13, the light source 1 is lit with 30% intensity, and the light source 2 is lit with 100% intensity.
In such a case, the dichroic mirror 6 combines the light L5 transmitted through the dichroic mirror 5, among the light L1 emitted from the light source 1, and the light reflected at the dichroic mirror 6, among the light L2 emitted from the light source 2, and emits this combined light L6 in the direction of the optical axis of the light L1.
The light intensity ratio of the light sources may be arbitrarily changed in accordance with the subject to be examined, the brightness of the screen, etc.

With the illumination apparatus according to this embodiment, in addition to the advantages achieved by the first embodiment described above, sharp band catting is possible by transmitting the light L1, which is emitted from the light source 1, through two dichroic mirrors. In this way, sufficient band separation is possible even with a filter with few dielectric layers (i.e., inexpensive filter).

### Third Embodiment

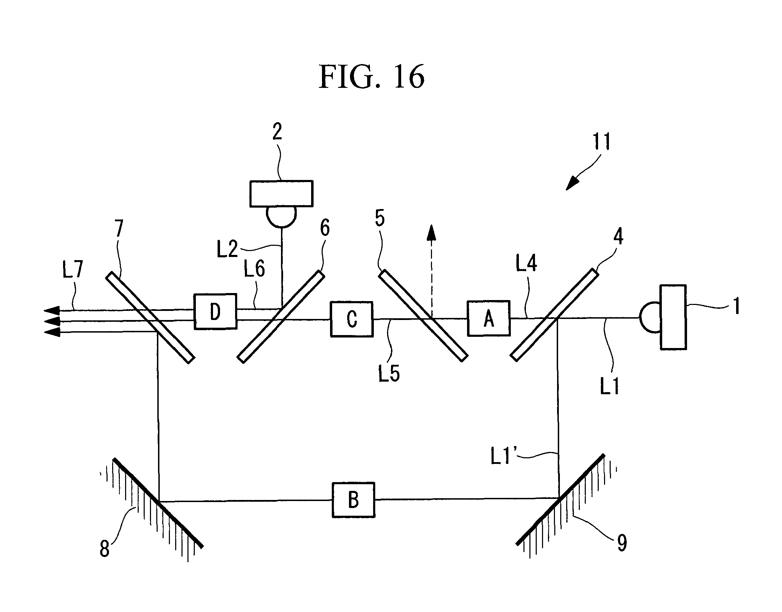

Next, an illumination apparatus according to a third embodiment of the present invention will be described mainly with reference to Figs. 16 to 27.
The illumination apparatus according to this embodiment differs from the embodiments described above in that light from the light sources are first separated and then recombined. For the illumination apparatus according to this embodiment, descriptions of the commonalities with the first embodiment are omitted, and mainly the differences are described below.

As illustrated in Fig. 16, a light source unit 11 includes a light source (first light source) 1 and a light source (second light source) 2, which are disposed such that the optical axes thereof are orthogonal to each other, dichroic mirrors 4, 5, 6, and 7 disposed on the optical axis of the light source 1, and mirrors 8 and 9 disposed on a branching optical path branched by the dichroic mirror 4.

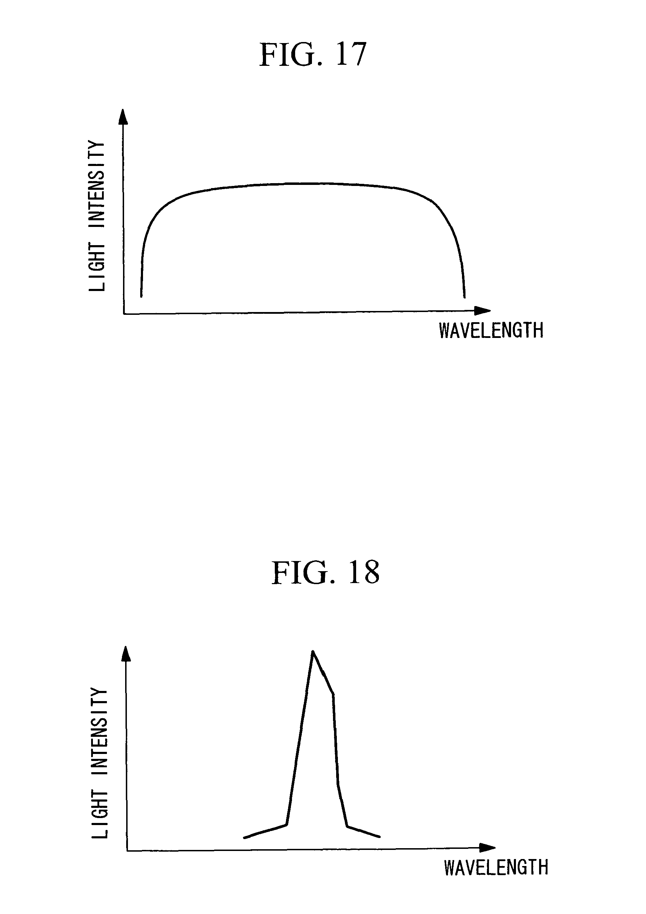

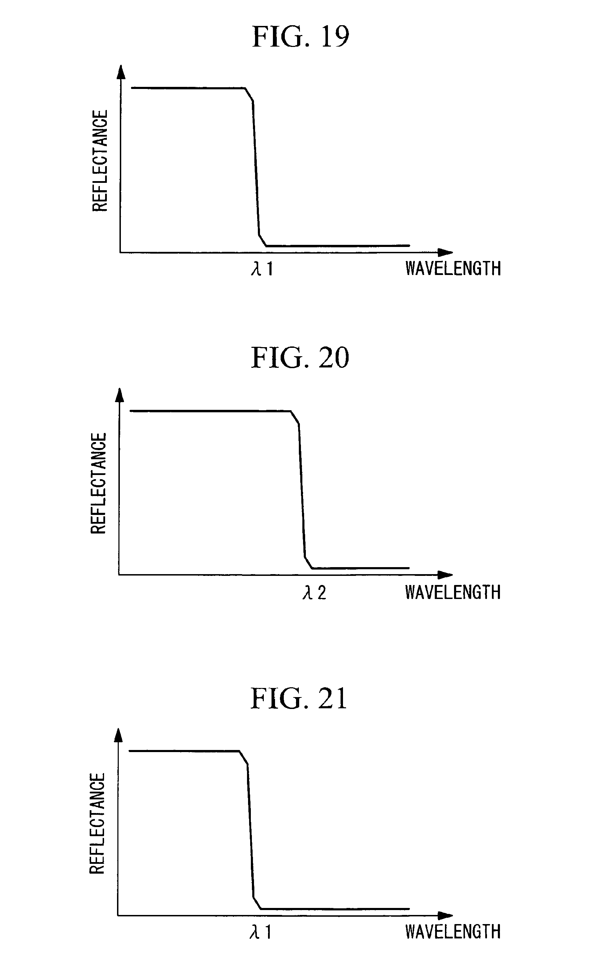

As illustrated in Fig. 17, the light source 1 is a light source that emits white light L1 in a wide wavelength band.
As illustrated in Fig. 18, the light source 2 is a light source that emits light L2 in a narrow wavelength band, such as a laser beam, and emits narrow-band light having substantially all components in an extremely limited wavelength band (wavelength band of wavelengths λ1 to λ2 illustrated in Figs. 19 and 20) compared with the light L1.

The dichroic mirrors 4, 5, 6, and 7 are disposed along the optical axis of the light source 1 in this order from near the light source 1.
As illustrated in Fig. 19, the first dichroic mirror 4 has a reflection characteristic in which light in a wavelength band larger than or equal to wavelength λ1 is transmitted, and light in the wavelength band smaller than wavelength λ1 is reflected.

As illustrated in Fig. 20, the second dichroic mirror 5 and the third dichroic mirror 6 have a reflection characteristic in which light in the wavelength band larger than or equal to wavelength λ2 is transmitted, and light in the wavelength band smaller than wavelength λ2 is reflected.
As illustrated in Fig. 21, the fourth dichroic mirror 7 has a reflection characteristic in which light in a wavelength band larger than or equal to wavelength λ1 is transmitted, and light in the wavelength band smaller than wavelength λ1 is reflected.

With the illumination apparatus having the above-described configuration, the light source 1 and the light source 2 are lit in the white light mode in which the subject S is irradiated with white light.
In such a case, the first dichroic mirror 4 transmits light L4 of wavelength λ1 or larger (optical path A), among the light L1 emitted from the light source 1, and emits light L1' below wavelength λ1 toward the mirror 9 (optical path B).

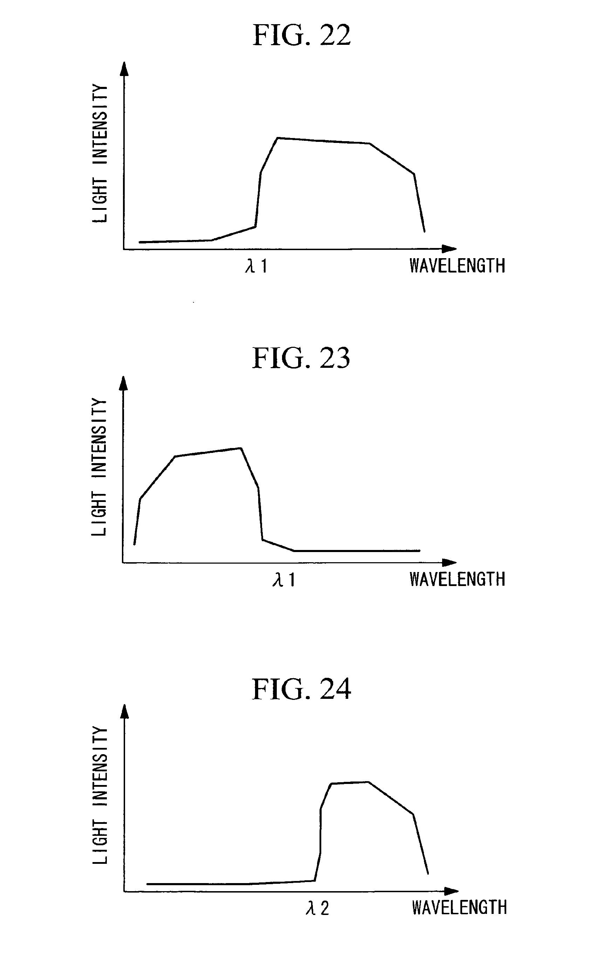

As illustrated in Fig. 22, the light L4 traveling along through the optical path A is light in a wavelength band larger than or equal to wavelength λ1, among the light L1 emitted from the light source 1.
As illustrated in Fig. 23, the light L1' traveling along the optical path B is light in a wavelength band smaller than wavelength λ1, among the light L1 emitted from the light source 1. The light L1' traveling along the optical path B is reflected to the fourth dichroic mirror 7 by the mirror 9 and the mirror 8.

The second dichroic mirror 5 transmits light L5 of wavelength λ2 or larger (optical path C), among the light L4 transmitted through the first dichroic mirror 4, and reflects light smaller than wavelength λ2 away from the optical path.
As illustrated in Fig. 24, the light L5 traveling along the optical path C is light in the wavelength band larger than or equal to λ2, among the light L4 transmitted through the first dichroic mirror 4.

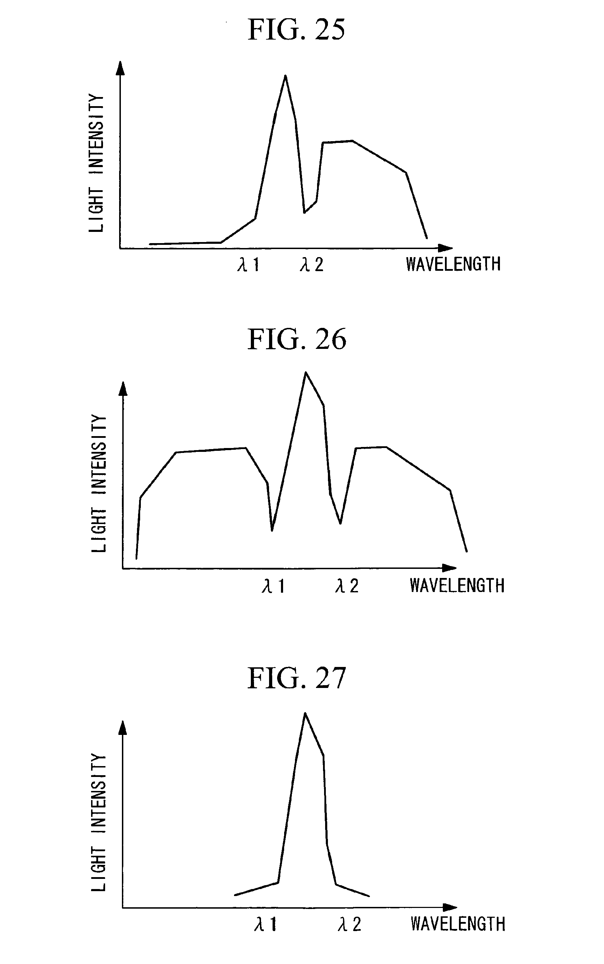

The third dichroic mirror 6 transmits light of wavelength λ2 or larger, among the light L5 transmitted through the second dichroic mirror 5, and reflects the light smaller than wavelength λ2, among the light L2 emitted from the light source 2. In this way, the third dichroic mirror 6 combines the light of wavelength λ2 or larger, among the light L5, and the light smaller than wavelength λ2, among the light L2, and emits the combined light L6 in the direction of the optical axis of the light source 1 (optical path D). The light L6 traveling along the optical path D is light having a spectral characteristic such as that illustrated in Fig. 25.

The fourth dichroic mirror 7 transmits light of wavelength λ1 or larger, among the light L6 transmitted through the third dichroic mirror 6, and reflects the light smaller than wavelength λ1, among the light L1' reflected at the mirror 9, in the direction of the optical axis of the light source 1. In this way, the fourth dichroic mirror 7 combines the light of wavelength λ1 or larger, among the light L6, and the light smaller than wavelength λ1, among the light L1', and emits the combined light L7 in the direction of the optical axis of the light source 1. In this way, the combined light L7 emitted from the fourth dichroic mirror 7 is light having a spectral characteristic such as that illustrated in Fig. 26.

In a special light mode in which the subject S is irradiated with special light, for example, the light source 1 is turned off, and only the light source 2 is lit.
In such a case, the light L6 traveling along the optical path D is light in a wavelength band smaller than wavelength λ2, among the light L2 emitted from the light source 2. The combined light L7 emitted from the fourth dichroic mirror 7 is light in a wavelength band larger than or equal to wavelength λ1, among the light L6 traveling along the optical path D. That is, as illustrated in Fig. 27, the combined light L7 emitted from the fourth dichroic mirror 7 is light in a wavelength band of wavelengths λ1 to λ2, among the light L2 emitted from the light source 2.

With the illumination apparatus according to this embodiment, in addition to the advantages achieved by the first embodiment described above, for example, light of an intermediate wavelength, such as green, can be cut with a lowpass filter and a high-pass filter, not a band-pass filter. In this way, the wavelength where folding of the a reflection characteristic occurs in the infrared region and ultraviolet region can be extended more than a band-pass filter, and thus, near-ultraviolet rays, near-infrared rays, etc., can be used as special light.

In this embodiment, it is described that, in the special light mode, the light source 1 is turned off and only the light source 2 is lit; instead, the light source 2 may be turned off and only the light source 1 may be lit. Moreover, similar to the second embodiment, the light intensity ratio of the light sources may be changed.

The embodiments of the present invention have been described above in detail with reference to the drawings. The detailed configuration, however, is not limited to the embodiments, and design changes etc., may also be included so long as they do not depart from the scope of the invention.
For example, in the first embodiment, an example in which the illumination apparatus according to the present invention is applied to an examination system has been described. Examples of the systems to which such an illumination apparatus can be applied include an endoscope system using special light (infrared light examination or chemical fluoroscopy) or a microscope system performing fluoroscopy.
Moreover, in the embodiments, an illumination apparatus including two light sources is described; instead, three or more light sources may be provided. Furthermore, a combination of multiple light sources emitting light of different wavelengths may be used as one light source. In this way, by using a combination of multiple light sources as one light source, illumination light having a desired wavelength can be easily provided.

### {Reference Signs List}

- 1: light source (first light source)
- 2: light source (second light source)
- 4: dichroic mirror

- 5: dichroic mirror (light combining unit)
- 6: dichroic mirror (light combining unit)
- 7: dichroic mirror (light combining unit)
- 10: illumination apparatus
- 11: light source unit (irradiation optical system)
- 12: light-source driving unit
- 13: illumination control unit (control unit)
- 21: image acquisition unit (light receiving unit)
- 22: image processing unit
- 23: image monitor
- 29: illumination-mode selecting unit
- 100: examination system
- S: subject

## Claims

1. An illumination apparatus configured to illuminate an illumination area in a plurality of illumination modes constituted of light in different predetermined bands,
having bands in which a first band required by one illumination mode of the plurality of illumination modes is narrower than a second band required by another illumination mode,
the apparatus comprising:
a first light source configured to emit illumination light in a band substantially the same as the first band;
a second light source configured to emit illumination light in at least the second band;
a light combining unit configured to transmit light in the first band, among the illumination light emitted from the first light source, in the direction of the illumination area as transmitted light while reflecting light outside the first band, among the illumination light emitted from the second light sources, in the direction of the illumination area as reflected light and to optically combine the transmitted light and the reflected light;
an illumination-mode selecting unit configured to select an illumination mode from the plurality of illumination modes;
and
a control unit configured to control the light intensity of light emitted from the first light source and the second light source on the basis of the selected illumination mode,
wherein the one illumination mode is a first illumination mode for lighting only the first light source, and the other illumination mode is a second illumination mode for lighting both the first light source and the second light source.

2. An examination system comprising:
the illumination apparatus according to Claim 1;
an irradiation optical system configured to irradiate a subject with light combined at the light combining unit; and
a light receiving unit configured to receive light reflected at the subject,
wherein the control unit controls the emission light intensity of the first light source and the second light source on the basis of the intensity of the reflected light received by the light receiving unit.

3. The examination system according to Claim 2, wherein the light receiving unit is an image acquisition element configured to acquire an image of the subject, and
the control unit controls the emission light intensity of the first light source and the second light source so as to set the brightness of the image acquired by the image acquisition element substantially constant.

4. An illumination apparatus configured to illuminate an illumination area in a plurality of illumination modes constituted of light in different predetermined bands,
having bands in which a first band required by one illumination mode of the plurality of illumination modes is narrower than a second band required by another illumination mode,
the illumination apparatus comprising:
a first light source configured to emit illumination light in a band substantially the same as the first band;
a second light source configured to emit illumination light in at least the second band;
a light combining unit configured to reflect light in the first band, among the illumination light emitted from the first light source, in the direction of the illumination area as reflected light while transmitting light outside the first band, among the illumination light emitted from the second light sources, in the direction of the illumination area as transmitted light and to optically combine the transmitted light and the reflected light;
an illumination-mode selecting unit configured to select an illumination mode from the plurality of illumination modes;
and
a control unit configured to control the light intensity of light emitted from the first light source and the second light source on the basis of the selected illumination mode,
wherein the one illumination mode is a first illumination mode for lighting only the first light source, and the other illumination mode is a second illumination mode for lighting both the first light source and the second light source.

5. An examination system comprising:
the illumination apparatus according to Claim 4;
an irradiation optical system configured to irradiate a subject with light combined at the light combining unit; and
a light receiving unit configured to receive light reflected at the subject,
wherein the control unit controls the emission light intensity of the first light source and the second light source on the basis of the intensity of the reflected light received by the light receiving unit.

6. The examination system according to Claim 5, wherein
the light receiving unit is an image acquisition element configured to acquire an image of the subject, and
the control unit controls the emission light intensity of the first light source and the second light source so as to set the brightness of the image acquired by the image acquisition element constant.
